# EUROPEAN PATENT APPLICATION

(11) **EP 1 435 387 A1**
(43) Date of publication of application: **07.07.2004**
(21) Application number: 01274525.3
(22) Date of filing: 12.12.2001
(51) Int. Cl.: C12N 1/21

(54) **THE ONCOLYTIC MICROORGANISMS EXPRESSING HSP AND USES THEREOF**

(30) Priority: 09.10.2001 CN 01141696
(71) Applicant: Hangzhou Conquer Biotech Co., Ltd., Hangzhou, Zhejiang Province 310012 (CH)
(72) Inventor: CHEN, Siyi, Pearland, TX 77584 (US); HU, Fang, Beijing 100081 (CN)
(74) Representative: Zounek, Nikolai, Dipl.-Ing.
(86) International application number: PCT/CN2001/001616
(87) International publication number: WO 2003/031602

(57) **Abstract**

The present invention is directed to the oncolytic microorganisms expressing Heat Shock Protein, compositions or pharmaceutical compositions containing them, and to methods of kilking local and metastatic tumors using them. The microorganisms refer to viruses and bacteria, which can grow selectively within tumor cells in order to lyse tumor cells. Upon the microorganisms or compositions according to the present invention is administrated to a tumor patient, the oncolytic microorganisms lye tumor cells and the heat shock proteins expressed by the oncolytic microorganisms or administered simultaneously with the oncolytic microorganisms adhere to tumor antigen released by the lysing tumor cells, and bind to antigen-presenting cells so as to induce specific immune response against tumor cells, then to kill metastatic tumors.

## Description

### TECHNICAL FIELD

The present invention relates generally to the field of tumor immunotherapy in biomedicine. Specifically, the present invention relates to a novel method of tumor therapy, that is, by using tumor antigens of cancer patients themselves to induce anti-tumor responses against their own local and metastatic tumors. The present invention also relates to a kind of oncolytic microorganism, a composition comprising the oncolytic microorganism and use of the oncolytic microorganism or composition in tumor therapy or pharmaceutical preparations to produce an anti-tumor drug.

### BACKGROUND OF THE INVENTION

At present, the routine tumor therapies are traditional surgery, radiotherapy and chemotherapy, all of which bring cancer patients great pain and burden. Moreover, in patients with metastases, it is rare to remove completely the tumors by surgery or to kill all the tumor cells by radiotherapy or chemotherapy. Excision of local tumor increases the risk of cancer metastasis in other parts of the patient's body and accelerates the spread of tumors.

Through changing the genetic characteristics, several viruses have been altered to infect cancer cells selectively. Some examples are herpes simplex virus-1 such as HSV-1 and G207; adenoviruses such as ONYX-015 (i.e., dl1520), CN706 and CN787; Newcastle disease virus (NDV) such as 73-T; vesiculovirus; and reovirus such as reolysin.^{[1-3]} Although some of these viruses have significant anti-tumor activity and have proven relatively effective at reducing or eliminating tumors in clinical trials ^{[3]}, suppression of metastatic tumors is obviously absent. The reason is that although the viruses can be directly cytolytic to the tumor cells, thereby contributing to tumor remission, tumor-specific cytotoxic T cell (CTL) responses in human patients are characteristically absent.^{[4]} The absence of CTL immune responses occurs because most tumor antigens lie within the tumor cells and are rarely released, except for a small amount after necrosis. Thus, the uptake of tumor antigens by professional antigen-presenting cells (APCs) is inefficient. Although the presence of the virus could trigger tumor-specific immunity, by delivering the "danger signal" and by enhancing the presentation of tumor antigens to APCs after virus-mediated destruction of the tumor cells, the immune response is very limited and additional mechanisms would be needed to further enhance anti-tumor immune responses.

Cancer vaccination strategies have focused on the use of killed tumor cells or lysates delivered in combination with adjuvants or cytokines. It shows great progress in cancer therapy research that cancer vaccination can induce an immune response against tumors. However, in many cases, tumor antigens are weak immunogens, i.e., the immune response against tumor antigens is not of a sufficient magnitude to confer immunity. At present, the main cancer vaccinations include immunization with isolated tumor-specific antigens, or cell therapy with the fusion of tumor cells with dendritic cells in vitro. However, different tumor antigens appear in different cancer patients, and there is even variation seen in the antigens expressed by different tumors in the same patient. Moreover, at a certain stage of tumor development, there may be different tumor antigens in the tumor cells of the same patient. Because of these limitations, the present cancer vaccinations have many drawbacks: Since there are no tumor antigens common to many kinds of tumors, individual tumor antigen vaccine must be designed for different patient. This individual and complicated procedure must be carefully performed to avoid contamination, and is inefficient and costly. Because of the complexity, expense and inefficiency, individual cancer vaccines are difficult to apply in clinical trials.

In conclusion, conventional surgery, radiotherapy and chemotherapy cancer treatments hurt the patient's body and do not effectively kill metastatic tumors. Gene therapy with a virus vector is only effective for local tumors. Passive immunotherapy with interleukins and interferons does stimulate immune-modulated responses, but tumor-specific CTL responses are characteristically absent. The novel individual "cancer vaccine" is expensive and not practicable yet. Therefore, until now there has been no anti-tumor drug that has high efficiency, low toxicity and a convenient means of administration. We propose such a system that can induce an efficient anti-tumor immune response against both local and metastatic tumors.

### SUMMARY OF THE INVENTION

The object of the present invention is to provide a novel method of tumor immunotherapy in order to use the tumor antigens of an individual patient to elicit an immune response of the body against local and metastatic tumors.

To achieve the object, the present invention provides a kind of oncolytic microorganism, which can specifically grow and replicate in tumor cells and express the protein that chaperones the antigens of tumor cells. In the following, the protein may be designated "antigen chaperon".

To achieve the object, the present invention provides a kind of APC, which is transfected with a vector that contains a DNA sequence encoding the antigen chaperon or fragment thereof.

To achieve the object, the present invention provides a kind of microorganism composition, including:
a) an oncolytic microorganism that can specifically replicate in and lyse tumor cells; and
b) a vector that can express the protein and fragment thereof that can chaperon antigens of tumor cells to APCs;
wherein the oncolytic microorganism can further contain a DNA sequence encoding a molecule that can enhance immune function.

To achieve the object, the present invention provides a kind of pharmaceutical composition that mainly contains:
a) oncolytic microorganisms that can selectively replicate in tumor cells; and
b) a protein or fragment thereof that can chaperon antigens of tumor cells to APCs; and
c) optionally containing immune-enhancement factor, immunologic adjuvant or pharmaceutical carrier, wherein the immune-enhancement factor may be expressed by the oncolytic microorganism.

To achieve the object, the present invention provides a kind of cancer immunotherapeutic agent, which includes the above said oncolytic microorganism, or the APCs, or the microorganism composition, or the pharmaceutical composition.

To achieve the object, the present invention provides a novel method of tumor therapy where the APCs are transfected with a vector that contains a DNA sequence encoding the protein or fragment thereof that can chaperon antigens of tumor cells to APCs.

To achieve the object, the present invention provides a method of tumor therapy that comprises administration to a cancer patient an immuno-efficient amount of said oncolytic microorganism, or the APCs, or the microorganism composition, or the pharmaceutical composition, or the immunotherapeutic agent.

In a preferred embodiment of the invention, oncolytic microorganisms with immuno-efficient amounts of antigen chaperon are administrated to cancer patients. Specifically, the oncolytic microorganisms with antigen chaperon are the recombinant oncolytic microorganism that expresses the protein or fragment thereof that can chaperon antigens of tumor cells to APCs; or the oncolytic microorganism and the DNA plasmid encoding above said antigen chaperon or fragment thereof; or the oncolytic microorganism and the replication-incompetent vector that contains a DNA sequence encoding above said antigen chaperon or fragment thereof. The oncolytic microorganisms are oncolytic viruses or bacteria that can specifically grow and replicate in tumor cells. These kinds of oncolytic microorganisms can lyse and kill the tumor cells by rapid replication. Thereafter, the previously masked tumor antigens are released and a "danger" signal is given to the immune system. The synchronously expressed antigen chaperon can capture the released tumor antigens and transfer them to APCs, which induce an immune response against local and metastatic tumors. A complex formed by synchronously expressed antigen chaperon and the released tumor antigens can also stimulate APCs, to active the immune system of the patient and then reduce or eliminate local and metastatic tumors.

In another preferred embodiment of the invention, APCs transfected with a vector that contains a DNA sequence encoding the antigen chaperon or fragment thereof are applied. The antigen chaperon is the protein that can chaperon antigens of tumor cells to APCs. The APCs containing the vector encoding the antigen chaperon or fragment thereof are more sensitive to antigen, and the antigen chaperon expressed by APCs can capture antigen and transfer it to APCs. Thus, the intensified immune signal will stimulate the immune response against local and metastatic tumors.

In another preferred embodiment of the invention, the composition of oncolytic microorganism that selectively replicates in tumor cells and expresses the above antigen chaperon or variants thereof is administrated to cancer patients. The oncolytic microorganisms rapidly replicate in tumor cells, thus lysing and killing the tumor cells so that the tumor antigens are released. Meanwhile, the synchronously administrated antigen chaperon has an affinity with APCs, thus the antigen chaperon assembles around the APCs and intensifies the danger signal, increasing APC maturation to induce an immune response against local and metastatic tumors.

The oncolytic microorganisms of the invention are preferably oncolytic viruses and bacteria that selectively replicate in tumor cells. Some examples of oncolytic viruses are herpes simplex virus, adenoviruses, NDV, vesiculovirus, reovirus and other oncolytic viruses that can selectively replicate in tumor cells. Some examples of oncolytic bacteria are Salmonella, Bifidobacterium, Shigella, Listeria, Yersinia, and Clostridium,^{[21]} which can selectively replicate in tumor cells.

The antigen chaperon that can chaperon antigens of tumor cells to APCs is preferably heat shock protein (HSP), which is from mammalian animal or microorganisms. The mammalian species include but are not limited to human, non-human primates and rodent. The microorganisms include but are not limited to Mycoplasma tuberculosis, Mycoplasma leprae, Trypanosoma cruzi, and Plasmodium falciparum. The oncolytic microorganisms with the antigen chaperon gene can further contain a DNA sequence encoding an immune-enhancement factor such as a cytokine (e.g., IL-2, IL-12, TNF, IFN, G-CSF, GM-CSF), or a chemokine and immune co-stimulating molecule (e.g., B7), etc.

In the present invention, the oncolytic microorganisms, the APCs, the oncolytic microorganisms' composition, the pharmaceutical composition and the immunotherapeutic agent can be applied alone or in combination with other immune modulators such as cytokines, immunological adjuvants, and Chinese traditional medicine.

It is plausible that the oncolytic microorganisms of the present invention, the composition or pharmaceutical composition thereof, kill the metastatic tumor by one or more of the following kinds of approach: A) The oncolytic microorganisms can specifically replicate and grow in tumor cells, and then lyse the cells to release tumor cell antigens. The synchronously expressed HSPs chaperon the released tumor antigens, thus inducing maturation of the APCs (mainly the patient's own dendritic cells), and then activate the immune effect cells, such as natural killer cells, CTLs and T helper cells. B)The protein that can chaperon antigens of tumor cells to APCs has an affinity with APCs, thus the antigen chaperon assembles around the APCs, intensifies the danger signal, and increases APC maturation to induce an immune response against local and metastatic tumors. C) the APCs transfected with a vector that contains a DNA sequence encoding the antigen chaperon or fragment thereof are more sensitive to released antigens, and the antigen chaperon expressed by APCs can capture antigen and transfer it to APC. This intensified immune signal will stimulate the immune response against local and metastatic tumors.

The primary advantage of the present invention is to overcome the main shortcomings of "cancer vaccines" due to individual differences in tumor antigens. These shortcomings include the fact that autologous tumor cells of the patients have to be isolated in advance and be cultured and be serially processed in vitro. The present invention can conveniently and efficiently chaperon antigens of tumor cells of each cancer patient to the patient's own APCs in real-time, and induce the immune response no matter what the development stage is or what kinds of tumor antigen are present. That is, through the own APCs of the tumor patient, the specific tumor antigens are presented and processed timely and dynamically and thus induce a specific immune response against the specific tumor antigens. For example, the oncolytic microorganisms provided by the present invention can lyse the tumor cells, while at the same time the HSP expressed by the oncolytic microorganisms chaperones the released tumor antigens to APCs. These APCs then induce the immune effect through cells that include CTLs, which kill tumor cells specifically; T helper cells; and natural killer cells. The activated immune responses not only lyse cells of the local tumor, but also inhibit or even kill the metastatic tumor. Compared with tumor therapy of using only oncolytic viruses, which can only treat local tumors, the method of the present invention can treat not only local tumors but also metastatic tumors and thus overcomes the limitation of the tumor therapy of using only oncolytic viruses.

### BRIEF DESCRIPTION OF THE DRAWINGS

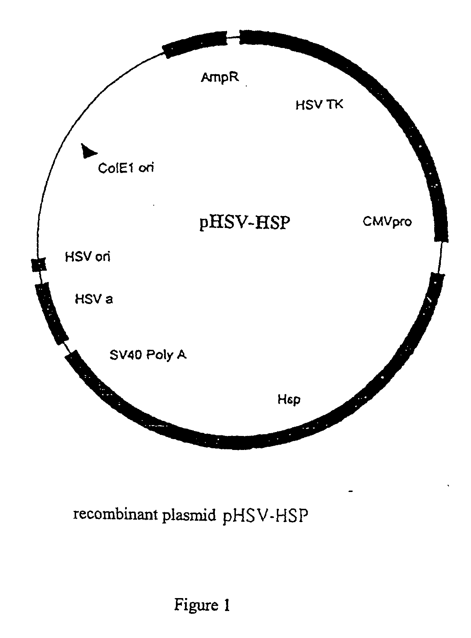

Fig. 1 is a schematic diagram of the constructed HSV-1 recombinant plasmid that expresses heat shock protein. The amplicon plasmid pHSV-HSP contains the HSP70 gene under control of the cytomegalovirus (CMV) promoter and simian virus 40 (SV40) polyadenylation sequence [SV40 poly(A)]. The amplicon plasmid also contains a selection marker; the origin of DNA replication of E. coli (ColE1ori); the HSV-1 cleavage/packaging signal (HSVα); and the HSV origin of DNA replication (HSVori).

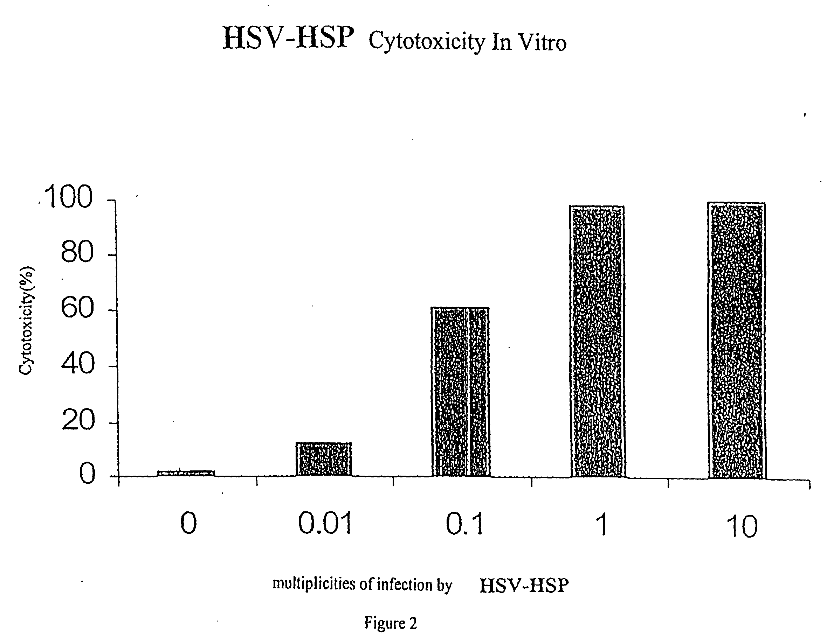

Fig. 2 is a diagram showing HSV-HSP cytotoxicity in vitro. CT26 mouse tumor cells (1x10⁵/well) were cultured on 24-well plates and infected with recombinant HSV-HSP or HSV viruses at different multiplicities of infection of 0.01 to 10. Cytotoxicity (%) was determined at four days post-infection by using a Promega kit, according to the manufacturer's instructions. When the multiplicity of infection was equal to 1, the cytotoxicity exceeded 99%.

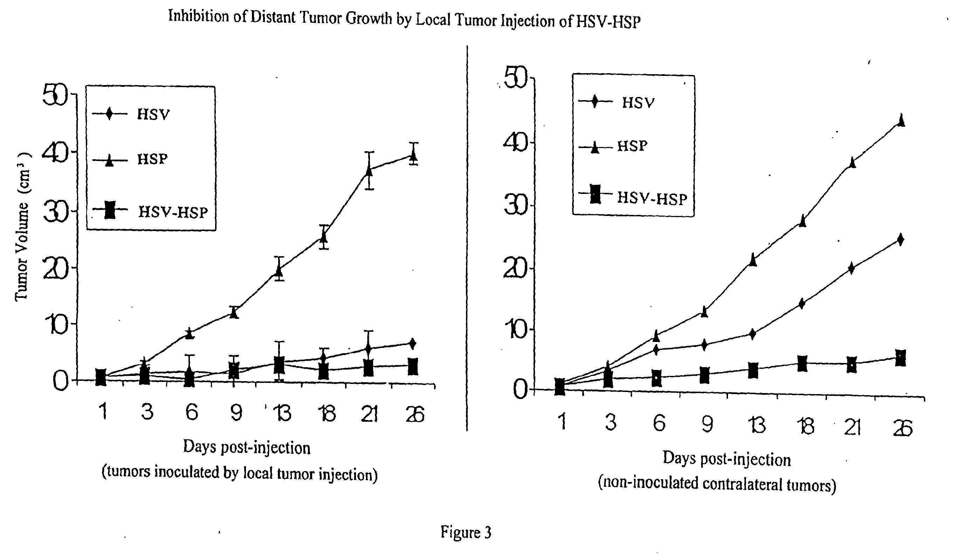

Fig. 3 shows that HSV-HSP has superior ability to inhibit distant tumor growth by local tumor injection. Syngeneic BALB/c mice containing bilateral CT26 tumors on each side of the rib region received inoculations in one tumor but not the other (contralateral) tumor. When bilateral subcutaneous tumors reached about 5 mm in maximal diameter, mice underwent unilateral intratumoral inoculation with HSV-HSP viruses (1x10⁶ plaque-forming units, PFU; "solid square"), HSV vector (1x10⁶ plaque-forming units, PFU; "solid diamond"), or HSP70 protein (Mock; "solid triangle") into the right side tumor on day 0, followed by a second inoculation on day 7 (n = 6/group). Both the inoculated tumors and their non-inoculated contralateral counterparts demonstrated significant tumor growth reduction after infection with HSV-HSP compared with Mock (p < 0.001 on day 25 post-infection; unpaired t test). Vaccination with HSV-HSP was more effective in inhibiting bilateral tumor growth than vaccination with either HSV or HSP protein (p < 0.001, on day 25 post-infection; unpaired t test).

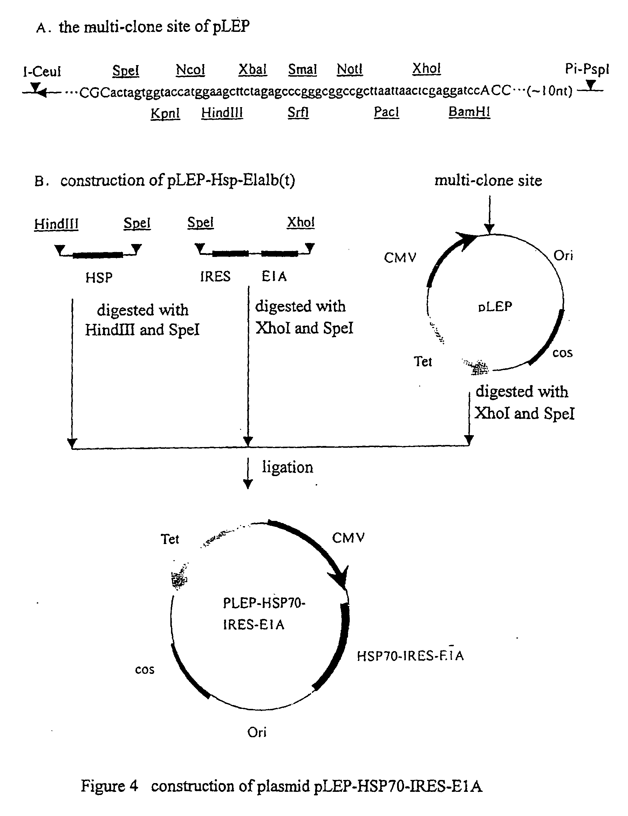

Fig. 4 is a schematic diagram of the constructed adenovirus plasmid pLEP-HSP70-IRES-E1A. To construct recombinant adenovirus using a double-plasmid method, the HSP gene and the E1A gene that is indispensable for the replication of adenovirus in tumor cells were ligated to the multi-clone site of plasmid pLEP. This generated the recombinant plasmid pLEP-HSP70-IRES-E1A.

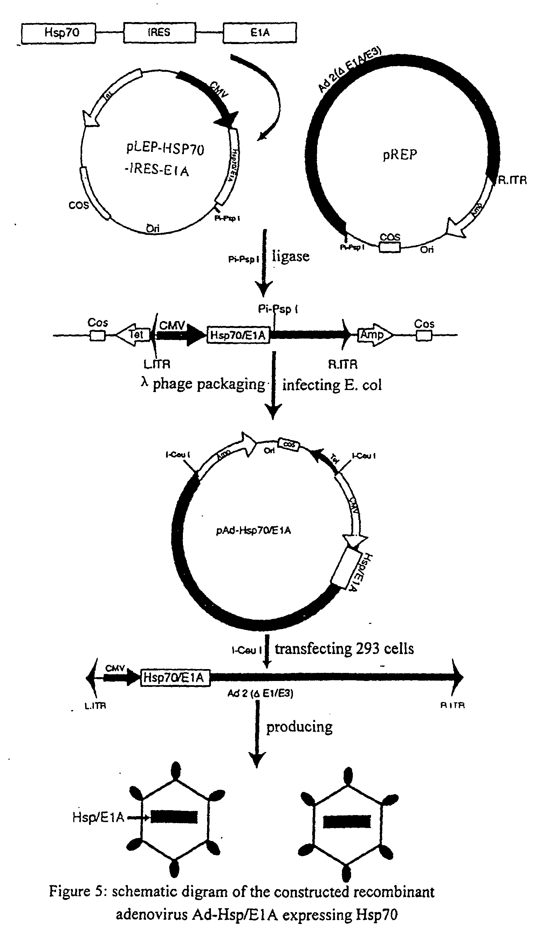

Fig. 5 is a schematic diagram of the constructed recombinant adenovirus Ad-HSP/E1A. The recombinant plasmid pLEP-HSP70-IRES-E1A was ligated to plasmid PREP, and the resulting product was packaged by phage y and the packaged product used to infect E. coli. The resulting recombinant plasmid pAd-Hsp70/E1A was cleaved by I-CeuI and then transfected into 293 cells to obtain a recombinant adenovirus that can express Hsp70 (Ad-HSP/E1A).

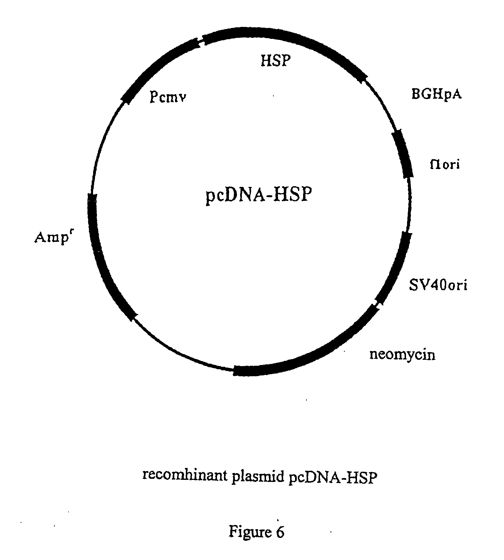

Fig. 6 is a schematic diagram of the constructed plasmid pcDNA-HSP that expresses heat shock protein. The HSP gene obtained from polymerase chain reaction (PCR) amplification was inserted into the eukaryotic expression vector pcDNA3.1 by blunt end ligation. The resulting recombinant plasmid pcDNA-HSP contains the HSP70 gene under control of the CMV promoter and the bovine growth hormone polyadenylation sequence [BGHpoly(A)], a selection marker, and the SV40 origin of DNA replication.

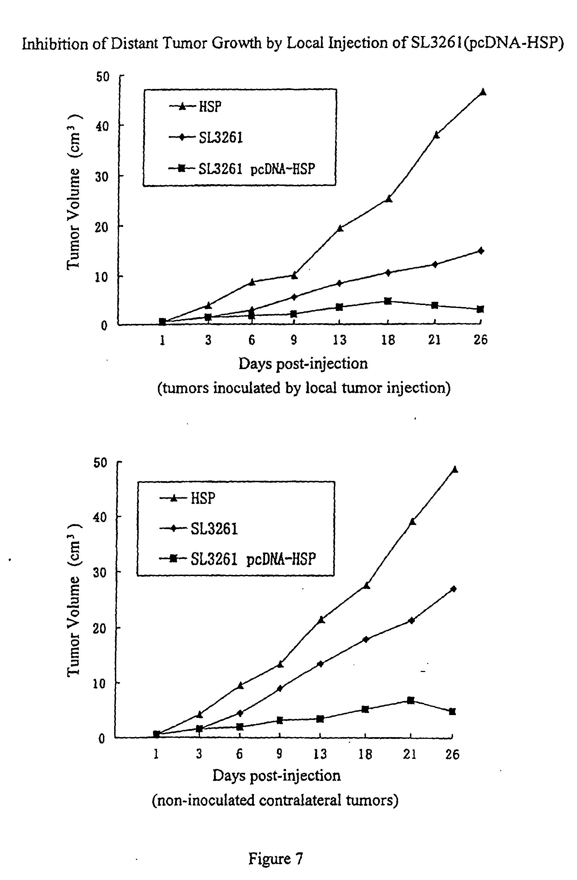

Fig. 7 shows that the Salmonella that can express HSP has superior ability to inhibit distant tumor growth by local tumor injection. Syngeneic BALB/c mice received inoculations with SMMC7721 on two sides of the rib region. When bilateral subcutaneous tumors reached about 5 mm in maximal diameter, mice underwent unilateral intratumoral inoculation with Salmonella containing the plasmid pcDNA-HSP (aroA-) (1x10⁷ PFU; "solid square"), or Salmonella not containing the plasmid (1x10⁷ PFU; "solid diamond"), or HSP70 protein (Mock control; "solid triangle") into the right side tumor on day 0, followed by a second inoculation on day 7 (n = 6/group). Both the inoculated tumors and their non-inoculated contralateral counterparts demonstrated significant tumor growth reduction after infection with Salmonella containing the plasmid pcDNA-HSP (aroA-) compared with Mock (p < 0.001 on day 25 post-infection; unpaired t test). Vaccination with Salmonella containing the plasmid pcDNA-HSP (aroA-) was more effective in inhibiting bilateral tumor growth than vaccination with either Salmonella not containing the plasmid or HSP protein (p < 0.001, on day 25 post-infection; unpaired t test).

### DETAILED DESCRIPTION OF THE INVENTION

Terms used in the context of the present invention have the general meanings that are known in the field. For clarification, certain terms used in the present invention are defined as follows:

The term "oncolytic microorganism" as used herein is defined as a microorganism that can enter tumor cells and selectively replicate in and destroy tumor cells. Examples of such microorganisms include oncolytic viruses and oncolytic bacteria.

The term "oncolytic virus" as used herein is defined as a virus that can replicate in and destroy tumor cells, regardless of p53 and other proteins with or without mutation. Examples of oncolytic viruses include replication-competent HSV-1, adenoviruses such as ONYX-015, NDV, vesiculovirus, etc.

The term "oncolytic bacterium" as used herein is defined as an engineered bacterium that can replicate in and destroy tumor cells. Some mutant bacterial strains can be applied as gene therapy vectors.^{[20]} For example, when the genes of pur or aroA in Salmonella are mutant, the bacteria cannot synthesize the products of these genes, which are indispensable for survival. In normal cells, these materials are absent, but altered genetic characteristics of tumor cells can result in these substances being present. Therefore, these dependent ("auxotrophic") bacterial stains are unable to survive in normal cells, but can selectively replicate in and lyse tumor cells. For example, two days after Salmonella typhimurium YS72(pur-) was inoculated intraperitoneally into mice with tumors, the ratio of bacteria present in tumor cells compared to normal liver cells was 9000:1.^{[20]} The HSP gene is introduced into the auxotrophic bacterial strain in the present invention, thus the bacteria can function as oncolytic bacteria because of their ability to flourish in tumor cells and cause the tumor cells to lyse.

The term "antigen chaperon" as used herein is defined as a kind of protein that can capture, attach or bind tumor-specific antigens to form a protein-antigen complex and then chaperon the antigen to APCs such as dendritic cells. The antigens are processed and presented to induce an immune response. Examples of the protein include molecular chaperon proteins such as heat shock protein.

The term "replication-incompetent vector" as used herein is defined as a microorganism, such as a virus, without the ability to replicate while still able to express a foreign protein.

The term "heat shock protein" as used herein is defined as a family of highly conserved molecules with ATPase activity. They are found in all prokaryotes and in most compartments of eukaryotic cells. HSP expression plays an essential role in protein metabolism under both stress and nonstress conditions, including being involved in de novo protein folding and membrane translocation and the degradation of misfolded expression constructs. HSP protein preparations, including hsp70, hsp90 and gp94/gp96 derived from tumor cells and virus-infected cells, are capable of eliciting cellular immunity.

The term "the variant of heat shock protein" as used herein is defined as the fragment or modifications of heat shock protein that include but are not limited to the addition, deletion or substitution of one or several amino acids, or a fusion protein combined with a foreign peptide. These kinds of modification can enhance the ability of HSP to chaperon antigens. Target localization sequences, such as signal peptides or nuclear localization sequences, can be joined to the HSP sequence to alter the distribution of HSP in the cell. For example, non-secretory HSP can be changed to secretory HSP, which can function outside the cell to bind antigen peptides efficiently.

The term "the plasmid that expresses heat shock protein" as used herein is defined as a plasmid DNA that can express HSP or variants thereof in vivo. The DNA expression sequence is placed in the proper context related to promoter, polyadenylation and enhancer sequences of the plasmid.

The term "replication-incompetent vector that contains a DNA sequence encoding the antigen chaperon or fragment thereof' as used herein is defined as the microorganism, such as virus, without the ability to replicate yet still able to express the foreign protein.

The term "adjuvant" as used herein is defined as a substance that can enhance immunity when combined with antigens, or that can act as an antigen itself.

The term "antigen" as used herein is defined as a molecule that provokes an immune response. This immune response may involve antibody production, the activation of specific immunological-competent cells, or both. An antigen can be derived from organisms, subunits of proteins/antigens, or killed or inactivated whole cells or lysates.

The term "cancer" as used herein is defined as a malignant cellular neoplasm (tumor) that invades other cells. Examples include but are not limited to breast cancer, prostate cancer, ovarian cancer, cervical cancer, skin cancer, pancreatic cancer, colorectal cancer and lung cancer.

The term "cancer vaccine" as used herein is defined as a tumor-specific molecule or immune cell that contacts tumor cells. The proteins that are expressed by tumor cells and make tumor cells immunogenic are tumor-specific antigens. Antigen proteins or epitopes are prepared in vitro, or immune cells can be incubated with tumor cells to make the immune cells recognize the antigens. The above said protein or peptide or immune cells can be applied as a therapeutic cancer vaccine.

The term "kill/killing tumor cells" or "destroy/destroying tumor cells" as used herein is defined as a process or result that the increase, filtration and metastasis of the tumor is efficiently inhibited or degraded or the tumor is changed to benign one.

The term "expression" as used herein is defined as the transcription and/or translation of a particular nucleotide sequence driven by its promoter.

The term "major histocompatibility complex," or "MHC," as used herein is defined as a specific cluster of genes, many of which encode cell surface proteins involved in antigen presentation. Among which, there are two types for determining histocompatibility. One is Class I MHC, or MHC-I, mainly functions in antigen presentation to CD8 T lymphocytes. The other is Class II MHC, or MHC-II, mainly functions in antigen presentation to CD4 T lymphocytes.

The term "promoter" as used herein is defined as the region of a nucleotide sequence that regulates transcription of a specific nucleotide sequence. The term promoter includes enhancers, silencers, and other cis-acting regulatory elements.

The term "T cell" as used herein is defined as a thymus-derived cell that participates in a variety of cell-mediated immune reactions.

The term "antigen-presenting cell," or "APC," as used herein is defined as a kind of cell whose function is to process and present antigens to T cells and B cells, including dendritic cells and macrophages.

The term "immune-enhancement factor" as used herein is defined as a molecule that can enhance immunity. Examples include cytokines, such as IL-2, IL-12, TNF, IFN, G-CSF, and GM-CSF; chemokines and immune co-stimulating molecules, such as B7, etc.

The term "immuno-efficient amount" as used herein is defined as the pharmaceutical dose that can induce sufficient immune response to suppress or kill tumor cells.

In this invention, our strategy is to use autologous tumor antigens and APCs to accomplish tumor immune therapy in vivo without the need for preparation of individual tumor vaccine or advance knowledge of the individual-specific tumor antigen of the patients.

To achieve the object of the present invention, we provide oncolytic microorganisms with a antigen chaperon gene that can induce a specific anti-tumor immune response. When the oncolytic microorganism is applied to patient tumors, it can lyse tumor cells, as well as stimulate APCs to induce an immune response that suppresses and kills the tumor metastases.

In one specific embodiment, the present invention provides a recombinant oncolytic microorganism that can express an antigen chaperon, which can chaperon antigens of tumor cells to APCs. The oncolytic microorganism can enter and replicate in tumor cells but not in normal cells, whilst also expressing the antigen chaperon that can chaperon the tumor antigens to APCs. Because the oncolytic microorganisms replicate prolifically and cause lysis of tumor cells, many tumor antigens are released. These kinds of antigens bind with the antigen chaperon expressed by the oncolytic microorganisms to form an antigen-protein complex. The complex can further be recognized by APCs, which process the antigens and present them to specific CTLs, as well as activating helper T cells capable of modulating the immune response, and natural killer cells to enhance the immunity.

In another specific embodiment of the present invention, the oncolytic microorganisms are applied in combination with the antigen chaperon that can chaperon antigens of tumor cells, or vectors that can express the antigen chaperon to achieve the object of this invention. The microorganisms can selectively replicate in and lyse tumor cells to release tumor cell antigens. These tumor cell antigens can be bound by the antigen chaperon to form a protein-antigen complex, then chaperoned to APCs such as dendritic cells, which further activates immune effect cells such as natural killer cells, CTLs and helper T cells to stimulate the specific immune response. Meanwhile, the antigen chaperon has an affinity with APCs, thus the antigen chaperon assembles around the APCs and intensifies the danger signal. This increases APC maturation to induce a specific immune response against tumors.

In another specific embodiment of the present invention, APCs transfected with a vector that contains a DNA sequence encoding the antigen chaperon or fragment thereof are applied. The object of the present invention can also be achieved by transfection of the APCs in vitro and application of the transfected APCs to the patients. The transfected APCs are more sensitive to released antigens. The antigen chaperon expressed by APCs can chaperon antigens to APCs and further intensify the immune signal to activate specific immunity against tumors.

To sum up, in addition to the oncolytic microorganisms that can express the antigen chaperon or the fragment thereof that can chaperon antigens of tumor cells to APC, the present invention includes but is not limited to the following compositions;

One composition includes:
a) an oncolytic microorganism that can specifically replicate in and lyse tumor cells; and
b) a vector that can express the protein and fragment thereof that can chaperon antigens of tumor cells to APCs.

Another composition includes:
a) an oncolytic microorganism that can selectively replicate in tumor cells; and
b) a replication-incompetent vector that contains a DNA sequence encoding above said antigen chaperon or fragment thereof.

Another composition includes:
a) an oncolytic microorganism that can selectively replicate in tumor cells; and
b) the protein and fragment thereof that can chaperon antigens of tumor cells to APCs.

The microorganisms can further contain DNA sequences that encode immune-enhancement factors, for example, cytokines such as IL-2, IL-6, IL-12, TNF, IFN, G-CSF, and GM-CSF; chemokines and immune co-stimulating molecules such as B7; and so on. The microorganisms or the microorganism composition of the present invention can be applied alone, or combined with other kinds of immunotherapeutic agent such as cytokines, adjuvant or Chinese traditional medicines. The microorganism composition can further contain pharmaceutical carrier.

The "antigen chaperon" or "the protein that can chaperon antigens of tumor cells to antigen-presenting cells" of the present invention is the protein that has an affinity with APCs, for example, the molecular chaperones, of which HSP is an example. HSP can bind tumor-specific antigens to form a protein-antigen complex and then chaperon the antigen to APCs such as dendritic cells. The antigens are processed and presented to APCs, which then further activates immune effect cells such as natural killer cells, CTLs and helper T cells to stimulate the specific immune response.

In a specific embodiment, we employed a microorganism expressing HSP to infect a local tumor in situ. It is found that the HSP expressed in the local tumor combines with tumor antigens, acts as a cancer vaccine and then elicits an immunomodulatory effect. The local expression of HSP can significantly enhance the anti-tumor activity of immune response. As a result, while the local tumor is killed or destroyed, metastatic tumors in a patient are also significantly suppressed.

Heat shock proteins are a family of highly conserved molecules with ATPase activity. They are found in all prokaryotes and in most compartments of eukaryotic cells. HSP expression plays essential roles in protein metabolism under both stress and non-stress conditions, including functions in de novo protein folding and membrane translocation, and the degradation of misfolded expression constructs.^{[5]} HSP proteins, including hsp70, hsp90 and grp94/gp96 derived from tumor cells and virus-infected cells, are capable of eliciting cellular immunity.^{[6-7]} The HSP can come from pathogen microorganisms including Mycoplasma tuberculosis, Mycoplasma leprae, Trypanosoma cruzi, Plasmodium falciparum; and other species such as primate, rodent, etc.

The immunogenicity of HSP proteins has been attributed to peptides bound to HSP expression constructs.^{[8]} The complex formed with HSP and antigenic polypeptide actives a specific cell which does not express endogenetic antigens. Then, the activated specific cell presents the antigen to T lymphocytes and thus stimulates the immune system of the tumor patient. These results demonstrate that HSP chaperones antigenic polypeptides to APCs, mainly dendritic cells, potentially allowing peptides to enter the MHC class I and II pathways for loading onto MHC class I and II molecules, where antigenic polypeptides can further activate cytotoxic T cells, natural killer cells and helper T cells to elicit a specific immune response.

All kinds of HSP or fragments thereof, or all kinds of variants thereof obtained by modifications (including but not limited to the addition, deletion or substitution of one or several amino acids), can be applied to carry out the present invention without departing from the scope and spirit of the invention, provided that the HSP or fragment or variants thereof can bind antigens.

In the specific embodiment of the present invention, in order to enhance the ability of HSP to chaperon antigens, modifications such as the addition, deletion or substitution of one or several amino acids, and fusion proteins combined with foreign peptides can be introduced. The HSP sequence can be placed in the context of target localization sequences such as signal peptides or nuclear localization sequences to alter the distribution of HSP in the cell. For example, non-secretory HSP can be changed to secretory HSP, which can be distributed outside the cell in order to bind antigen peptides efficiently.

The oncolytic microorganisms of the invention are preferably natural or genetically modified oncolytic viruses and bacteria that selectively replicate in tumor cells. Some examples of oncolytic viruses are HSV such as HSV-1 and G207; adenoviruses such as ONYX-015 (i.e., dl1520), CN706 and CN787; NDV such as 73-T; vesiculovirus; reovirus such as reolysin^{[1-3]}; and other oncolytic viruses that can selectively replicate in tumor cells. The bacteria include Salmonella, Bifidobacterium, Shigella, Listeria, Yersinia, and Clostridium^{[21]} and other kinds of bacteria that can selectively replicate in tumor cells in their natural environment or by mutation. The oncolytic microorganisms may include variants of above said virus or bacteria, provided that the microorganisms can selectively replicate or grow in tumor cells and cause lysis of the tumor cells.

To achieve the object of the present invention, the oncolytic microorganisms, such as HSV-1 or d11520, are applied with the gene encoding a protein, such as HSP, that can chaperon antigens of tumor cells to APCs. It is readily apparent to one skilled in the art that the gene encoding a protein such as HSP can be applied in the present invention if the gene is correctly ligated with regulation sequences, such as promoter and terminator sequences, no matter whether the gene is integrated into the genome of the oncolytic microorganism or is free from the genome. For example, the gene may be presented in other expressing vectors contained in the oncolytic microorganism. It is also obvious that various embodiments and modifications may be made to the invention disclosed in this Application without departing from the scope and spirit of the invention.

In a preferred embodiment, a recombinant oncolytic adenovirus expressing HSP70 Ad-HSP/E1A was generated. The HSP70 gene was incorporated into a d11520 that can selectively replicate in p53-defective tumor cells ^{[9-14]}, as described in Example 2. In the mouse tumor models, the recombinant adenovirus expressing HSP70 Ad-HSP/E1A was shown to have potent anti-tumor activity (see table below). The experiment shows that the recombinant adenovirus can effectively reduce the tumor size or eliminate the tumor. The experimental results from combined use of oncolytic adenoviruses with HSP70 proteins or HSP70 expression DNA or replication-defective HSP70 expression viruses are also listed in the table below:

| | Group 1 | | Group 2 | Group3 | | Group 4 |
|---|---|---|---|---|---|---|
| Tumor model | Secreted | Non-secreted | | Secreted | Non-secreted | |
| CT26/Balb/c | | | | | | |
| TRAMP-c2/c57 | | | | | | |
| B16/C57 | | | | | | |
| Table Notes: Group 1: Ad-Hsp/E1A, the recombinant oncolytic adenovirus expressing HSP70 of the present invention Group 2: A composition of oncolytic adenovirus Ad-ΔE1B and Hsp70 expression vector plasmid DNA Group 3: A combination of oncolytic adenovirus Ad-ΔE1B and replication-defective adenoviruses expressing Hsp70 Group 4: A combination of oncolytic adenovirus Ad-ΔE1B and Hsp70 proteins "CT26/Balb/c" represents the CT26 colon cancer model in Balb/c mice. "TRAMP-c2/c57" represents the TRAMP-c2 prostate cancer model in C57 mice. "B16/c57" represents the B16 melanoma model in C57 mice. | | | | | | |

Tumor cells (2x14⁵) were injected subcutaneously into the bilateral flanks of mice (right side and left side). These mice are regarded as animal models suffering from metastatic tumors. When the subcutaneous tumors were palpably growing (about 8-10 days after tumor inoculation), each animal then underwent a unilateral intratumoral inoculation (right flank) of Ad-Hsp/E1A about 2x10⁹ PFU; or a composition comprising Ad-ΔE1B about 2x10⁹ PFU and Hsp70 expression vector DNA (50 µg); or a combination of oncolytic virus Ad-ΔE1B 2x10⁹ PFU and a replication-defective Ad-HSP70 about 2x10⁹ PFU; or a composition comprising oncolytic Ad-ΔE1B about 2x10⁹ PFU and HSP70 about 10-25 µg. Tumor size was measured by external caliper every 3-4 days. The preliminary results are shown in the Table. " "rep resents the results that showed inhibitory activity toward the treated tumor as well as toward the contralateral, untreated tumors. " "represents the experiments that are not yet finished.

In a specific embodiment, the oncolytic virus is HSV. A replication-competent HSV mutant has been found to be nonpathogenic in a number of animal models and is progressing toward clinical trials for the treatment of primary human brain tumors.^{[16]} This mutant HSV replicates in dividing cells with resultant cell death, whereas its growth in non-dividing cells is highly attenuated. Inoculation of established tumors in mice with HSV leads to inhibition of local tumor growth and prolonged animal survival due to tumor-selective replication.^{[15-17]} An amplicon plasmid encoding HSP70 is introduced into the HSV viruses to construct the recombinant HSV, using either of the construction methods described below.

One construction method: The cDNA for human HSP70 was amplified using PCR from a human cDNA library, filled-in with Klenow fragments and DNA-sequenced. The HSP70 DNA fragment was then inserted into the SpeI site of pHSV plasmid to generate the resultant vector pHSV-HSP. The vector and a HSP helper virus were co-transfected into host cells, and packaged to generate recombinant HSV viruses that express HSP proteins. Please see Example 1 for the detailed procedure for generation of the recombinant HSV.

Another construction method: A vector comprising a gene encoding HSP under the control of promoter was constructed. The vector was cloned into a HSV amplicon and then incorporated into the HSV genome by employing conventional means in the art. The resultant genetically recombinant HSV having the gene encoding the HSP integrated into the genome of the HSV could be directly used for treatment without the need for co-transfection of HSV helper and plasmid DNA.

In a preferred embodiment, the oncolytic virus is adenovirus. The adenovirus mutant that was used in this invention selectively replicates in tumor cells, and only weakly replicates in normal cells.^{[10]} This oncolytic adenovirus can selectively replicate in and inhibit tumor xenografts in mice and prolong the survival of the treated mice.^{[9-11]} The recombinant oncolytic adenovirus was generated by using a pLEP and pREP two-plasmid system. The HSP gene and Ad E1A gene which is necessary for an adenovirus to be able to replicate in tumor cells were cloned into the pLEP vector to generate pLEP-HSP70-IRES-E1A. The resultant vector and pREP were digested and ligated together and packaged into λ phages, which were then transfected into E. coli. The recombinant pAd-Hsp70/E1A cut with I-CeuI was then transfected into 293 cells to generate the recombinant oncolytic adenovirus that expresses HSP70. See the detailed procedure in Example 2.

In a preferred embodiment, a mutant of Salmonella typhimurium which is aroA-deficient and selectively grows in tumor cells that can provide the nutrients for the mutant of S. typhimurium to grow,^{[19]} was used to express HSP. The Salmonella typhimurium mutant selectively grows in and lyses tumor cells. The HSP proteins expressed by the Salmonella typhimurium combined with antigenic peptides that released from killed tumor cells to present antigens to APCs, leading to the activation of CTL-mediated immune responses and inhibition of tumor growth in mouse tumor models. The survival of the mice was prolonged. See Example 3 for detail.

In specific embodiments, the nucleic acid encoding a human hsp70 gene is under transcriptional control of a promoter in the oncolytic HSV. The "promoter" refers to a DNA sequence recognized by the synthetic machinery of the cell, or introduced synthetic machinery, required to initiate the specific transcription of a gene. Much of the thinking about how promoters are organized derives from analyses of several viral promoters, including those for CMV, the HSV thymidine kinase, and SV40 early transcription units. These studies, augmented by more recent work, have shown that promoters are composed of discrete functional modules, each consisting of approximately 7-20 bp of DNA, and containing one or more recognition sites for transcriptional activator or repressor proteins. Additional promoter elements, i.e., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase promoter, the spacing between promoter elements can be increased to 50 bp apart before activity begins to decline. Depending on the promoter, it appears that individual elements can function either cooperatively or independently to activate transcription.

A promoter may be one naturally associated with a gene or sequence, as may be obtained by isolating the 5' non-coding sequences located upstream of a coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other prokaryotic, viral, or eukaryotic cell, and promoters or enhancers not "naturally occurring," i.e., containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR. Furthermore, control sequences that direct transcription and/or expression of sequences within non-nuclear organelles such as mitochondria, chloroplasts, and the like, could be employed as well.

A promoter sequence exemplified in the experimental examples presented herein is the immediate early CMV promoter sequence. This promoter sequence is a strong constitutive promoter sequence capable of driving high levels of expression of any nucleotide sequence operatively linked thereto. However, other constitutive promoter sequences may also be used, including, but not limited to the SV40 early promoter, mouse mammary tumor virus (MMTV) promoter, human immunodeficiency virus long terminal repeat promoter, Moloney virus promoter, the avian leukemia virus promoter, Epstein Barr virus immediate early promoter, Rous sarcoma virus promoter, as well as human gene promoters such as, but not limited to, promoters from the actin, myosin, hemoglobin, and muscle creatine genes. Further, the invention should not be limited to the use of constitutive promoters. Inducible promoters are also contemplated as part of the invention. The use of an inducible promoter in the invention provides a molecular switch capable of turning on expression of the nucleotide sequence, which is operatively linked when such expression is desired, or turning off the expression when expression is not desired. Examples of inducible promoters include, but are not limited to, promoters that respond to metallothionin, glucocorticoid, progesterone, and tetracycline. Further, the invention includes the use of a tissue-specific promoter, where a promoter is active only in a desired tissue. Tissue-specific promoters are well known in the art and include, but are not limited to, the HER-2 promoter and the PSA-associated promoter sequences.

For expression of HSP, one will typically include a polyadenylation signal to effect proper polyadenylation of the transcript. The nature of the polyadenylation signal is not believed to be crucial to the successful practice of the invention, and any such sequence may be employed. Preferred embodiments include the SV40 polyadenylation signal, long terminal repeat polyadenylation signal, and/or the bovine growth hormone polyadenylation signal, all of which are convenient and/or known to function well in various target cells. It is also contemplated that a transcriptional termination site can be used to terminate transcription. These elements can serve to enhance message levels and/or to minimize read-through from the cassette into other sequences.

It is important to note that in the present invention it is not necessary for the oncolytic virus vector to be integrated into the genome of the host cell for proper protein expression. Rather, the expression vector may also be present in a desired cell in the form of an episomal molecule. For example, there are certain cell types in which it is not necessary that the expression vector replicates in order to express the desired protein. These cells are those that do not normally replicate, such as muscle cells, and yet are fully capable of gene expression. An expression vector may be introduced into non-dividing cells and express the protein encoded thereby in the absence of replication of the expression vector.

In the present invention, a patient receives the oncolytic microorganism combined with the protein that can chaperon tumor cell antigens, or the vector that can express the protein or with the APC transfected with the DNA sequence encoding the protein or fragment thereof to stimulate the specific immune response against local and metastatic tumors. The microorganism or the composition containing the oncolytic microorganisms can be applied alone or combined with other immunotherapeutic agents, such as cytokines or Chinese traditional medicine. In practice, an immune modulator such as adjuvant or immune-enhancement factor can be applied to enhance the immunity. A number of studies have shown that anti-tumor immune responses can be enhanced by co-administration of a cytokine-expressing plasmid or cytokine. A skilled artisan readily recognizes that the nucleotide sequences for a cytokine and the nucleotide sequences for HSP can be incorporated into one expression vector; thus eliminating the use of two separate vectors. Furthermore, a skilled artisan also knows that to increase the expression level of HSP, several copies of HSP gene can be incorporated into one expression vector.

Where appropriate, the oncolytic microorganism, microorganism composition or pharmaceutical composition can be formulated into an immunotherapeutic agent. These oncolytic microorganisms with functional genes, oncolytic microorganism compositions or pharmaceutical compositions can be formulated into preparations in solid, semisolid, liquid or gaseous forms in the ways known in the art for their respective routes of administration. Means known in the art can be utilized to prevent release and absorption of the composition until it reaches the target organ or to ensure controlled release of the composition. A pharmaceutically acceptable form should be employed which does not render ineffective the compositions of the present invention. In pharmaceutical dosage forms, the compositions can be used alone or in appropriate association, as well as in combination, with other pharmaceutically active compounds. A sufficient amount of viruses or bacterial containing a gene encoding chaperon proteins must be administered to provide a pharmacologically effective dose of the gene product. The oncolytic microorganism can be administered alone or formulated with various adjuvants.

Usually, the above said oncolytic microorganism, microorganism composition, pharmaceutical composition, or immunotherapeutic agent of the present invention, are administered in immuno-efficient amounts alone or in combination with other therapeutics as the conventional and acceptable means known in the art. The immuno-efficient amount varies depending on the degree of the illness, the healthy conditions, the age of the patients, the efficacy of the microorganisms, and other factors. In general, one skilled in the art can easily determine the immuno-efficient amount to be administered to tumor patients, according to the knowledge and the disclosure of the present invention.

The adjuvants capable to be used in combination with the microorganism of this invention include but are not limited to Freund's complete adjuvant, Freund's incomplete adjuvant, aluminum hydroxide, Bacillus Calmett-Guerin (BCG), lipid polysaccharide (LPS), Lipid A analog, muramyl dipeptide (MDP) and analog thereof, endotoxin (LT) and cholera toxin (CT).

The pharmaceutical compositions of this invention may be administered intratumorly, orally, systematically such as via intranasal, skin and suppository, or parenterally such as intramuscular injection, intravenous injection and subcutaneous injection. According to the conventional techniques known to those skilled in the art, the pharmaceutical compositions or the immunotherapeutic agents of this invention can be formulated with pharmaceutically acceptable carrier and/or vehicle. Non-limiting examples of the formulations include a tablet, a pill, a capsule, a semisolid, a granule, an extended-release dose, a solution, a suspension or an emulsion, an aerosol and other acceptable carriers. The oncolytic microorganisms are administered in combination with at least one kind of pharmaceutically acceptable excipient. The pharmaceutically acceptable excipient is an innoxious material that cannot substantially compromise the efficacy of the microorganisms and therapeutic effect of other active components or that may aid administration. The excipient can be solid, liquid, semisolid, or aeriform in an aerosol dose, all of which are easy to obtain for those skilled in the art.

The solid excipients include, but are not limited to, starch, cellulose, talc, glucose, lactose, saccharose, glutin, maltose, rice, flour, chalk, silica gel, magnesium stearate, sodium stearate, stearate glycerol, sodium chloride, skim milk powder, and so on. The liquid and semisolid excipients include, but are not limited to, water, ethanol, glycerol, propylene glycol and all kinds of oil including oils that come from petroleum, animal, plant oil such as from peanut, soy or sesame, or composed oil. The preferred liquid carriers are suitable for liquid injection, including water, salt solution, glucose solution, and diglycol.

The amount of the microorganism comprised in the pharmaceutical composition of this invention varies depending on the kind of pharmaceutical form, unit dosage, the kind of excipient, and other factors known by those skilled in the art. Furthermore, the actual dose and schedule can vary depending on whether the compositions are administered in combination with other pharmaceutical compositions, or depending on individual differences in pharmacokinetics, drug disposition, and metabolism. Furthermore, the amount of the virus vectors to be added per cell will likely vary with the length and stability of the therapeutic gene inserted in the vectors, as well as with the nature of the sequence. The amount of vectors is a parameter that needs to be determined empirically, and that can be altered due to factors not inherent to the methods of the present invention. One skilled in the art can easily make any necessary adjustments in accordance with the requirements of the particular situation.

### Examples

The present invention is described in detail by way of examples. These examples are illustrative and are not intended to limit the scope of the invention in any manner.

### Example 1

### Human HSP70 Gene Amplification and Cloning

Human cDNA was amplified from SKOV3 cells (ATCC HTB-77) by reverse transcriptase-PCR. The upstream primer was GGT ATG GAA GAT CCC TCG AGA TC and the downstream primer was TA CTA ATC TAC CTC CTC AAT GGT GGG. The PCR machine from Perkin-Elmer company was used, and the reaction conditions were as follows: the final concentration of primers were 30 pM, dNTP 100 mM, template DNA 100 ng, Taq DNA polymerase 2.5 U. Other reaction conditions were according to the instructions of the GeneAmp DNA kit, where each reaction volume was 100 µl. Each reaction was covered with 75µl mineral oil. Thirty amplification cycles were used, including denaturation at 92°C for 1 minute, annealing at 50°C for 1 minute, and extension at 72°C for 2 minutes. PCR products were then purified using Qiagen kits.

### Generation of Recombinant Oncolytic HSV

We have used a replication-competent, mutated herpes simplex virus type-1 (HSV-1),^{[15-18]} in which the 34.5 gene was deleted. This mutant HSV-1 replicates in dividing cells with resultant cell death, whereas its growth in non-dividing cells is highly attenuated.^{[16]} Inoculation of established tumors in mice with HSV leads to inhibition of local tumor growth and prolonged animal survival due to tumor-selective replication.^{[16]} The HSV mutant was used to deliver HSP70 into tumors. The cDNA for human HSP70 was PCR-amplified from a human cDNA library and DNA-sequenced. The PCR-amplified DNA was filled in using Klenow fragment and then inserted into the SpeI site of plasmid pHSV (Geller, A. I. and Breakefield, X. O. (1988), Science 241:1667-1669). The resultant plasmid is designated as pHSV-HSP (Fig. 1). The 34.5 gene-deleted HSV was used as helper to generate recombinant HSV-HSP viruses. The pHSV-HSP plasmid and 34.5 gene-deleted HSV were transfected into host cells to generate recombinant HSV-HSP viruses. With the helper virus HSV^{[17]}, the amplicon plasmid encoding HSP70 Phsv-HSP is packaged, thus a recombinant HSV virus expressing HSP 70 is produced. Each virus particle contains about 15 copies of the HSP70 gene that can transduce both dividing and non-dividing cells at high efficiency. The viral DNA does not integrate into the infected cell genome and with the CMV promoter driving HSP70, expression is strong but transient. The pHSV-HSP was sequenced and data regarding it are presented in the Sequence Listing.

### Titering of Recombinant HSV-HSP

Vero cells were cotransfected with purified amplicon plasmid DNA (pHSP70) and HSV viral DNA using lipofectAMINE (Life Technologies), as described by the manufacturer, and then cultured at 34.5°C until the cells exhibited complete cytopathic effect. Virus was then harvested from the Vero cells and passaged at a 1:5 dilution in Vero cells until inhibition of helper virus replication was observed. Generally, 5-6 passages were needed to observe the inhibition of helper virus replication. The HSP70-containing HSV virus was termed HSV-HSP. Recombinant virus stocks were titered after a freeze-thaw/sonication regimen and removal of cell debris by low speed centrifugation (2000 x g for 10 min at 4°C). HSV titer was expressed as the number of PFUs after a plaque assay on Vero cells at 34.5°C. For HSV-HSP, HSP70 expression was determined and the passage with the highest level of titre was used. The virus titers were about 5x10⁷ PFU/ml for HSV-HSP, and about 6x10⁷ PFU/ml for helper HSV.

### Oncolytic Activity of HSV-HSP Viruses In Vitro

Although HSV-1 replicates in a wide variety of tumor cell types, the murine colorectal carcinoma cell line CT26 was found to be susceptible to HSV infection. This cell line is poorly immunogenic and does not induce detectable tumor-specific CTLs. CT26 tumors are somewhat refractory to cytokine treatment. The immunodominant MHC class I-restricted Antigene for CT26 has been identified as a nonamer peptide derived from the envelope protein (gp70) of an endogenous ecotropic murine leukemia provirus. Adoptive transfer studies have established the correlation between induction of tumor-specific CTL and an anti-tumor effect on established subcutaneous CT26 tumors. The murine tumor cells CT26 were cultured in 24-well plate (1x10⁵ cells per well). Then the CT26 cells were infected with HSV-HSP or HSV at a multiplicity of infection from 0.01 to 10. Infection of CT26 cells with HSV-HSP or HSV results in death of 70% of the cells by 4 days post-infection at a multiplicity of infection of 0.1. Infection of HSV-HSP or HSV at a multiplicity of infection of 1 resulted in 99% cytotoxicity by 4 days post-infection (Fig. 2). The expression of HSP70 was detected by radio-labeling and immunoprecipitation, then SDS-polyacrylamide gel electrophoresis after infection of tumor cells in culture. These data indicate that the insertion of HSP70 does not reduce HSV replication and cytotoxicity.

### Inhibition of Distal Tumor Growth in Mouse Model

The anti-tumor efficacy of the combined HSV-HSP therapy was evaluated in the CT26 tumor model in syngeneic BALB/c mice, which were obtained from Charles River (Wilmington, MA, USA). All animal procedures were approved by the Animal Care and Use Committee. Mice were divided into three groups: groups 1 and 2 were for therapeutic tests and the third group was for control. CT26 tumor cells (1x10⁵) were injected subcutaneously into the bilateral flanks of mice. Those mice received inoculation of CT26 tumor cells were regarded as animal model suffering from metastatic tumors. When subcutaneous tumors were palpably growing (about 5 mm in maximal diameter), each animal of Group 1 then underwent a unilateral intratumoral inoculation of HSV-HSP stock (1x10⁶ PFU) in the right flank tumor, followed by a second inoculation 7 days later. Each animal of Group 2 then underwent a unilateral intratumoral inoculation of HSV-1 stock (1x10⁶ PFU) in the right flank tumor, followed by a second inoculation 7 days later. HSV-1 was used as a control for HSV-HSP inoculation so that differences in viral factors would be accounted for. To evaluate the effect of the HSP per se, each animal of Group 3 then underwent a unilateral intratumoral inoculation of HSP in the right flank tumor, followed by a second inoculation 7 days later. Tumor size was measured by external caliper, and tumor volume was calculated (V = h x w x d). If animals appeared moribund or the diameter of their subcutaneous tumors reached 18 mm, they were killed and this was recorded as the date of death for survival studies. Statistical differences were calculated using StatView 4.5 (Abacus Concepts, Berkeley, CA), where mean tumor volume was assessed by unpaired t test. Inoculation with HSV-HSP elicited a very prominent anti-tumor effect, with both the inoculated tumors and their non-inoculated contralateral counterparts demonstrating a significant reduction in tumor growth (Fig. 3). Upon injection of HSV-HSP, not only the -inoculated tumors but also the non-inoculated counterpart tumors (distal tumors) were reduced to an undetectable size. Inoculation with HSV only resulted in a significant reduction in tumor growth of inoculated tumors, but had less effect in non-inoculated tumor (Fig. 3). The control group injected with HSP demonstrated that HSP per se has little effect on reducing the tumor size of the inoculated tumors and non-inoculated tumors. These results indicate that the expression of HSP70 by HSV elicits potent anti-tumor immune responses to block distal tumor growth.

### Example 2

### Construction of recombinant adenovirus

The recombinant adenovirus is constructed by a two-plasmid system, pLEP and PREP Ad system.

The adenovirus type 2 E1 region from bases 559 to 2262 was generated by PCR using adenovirus Ad1055 wild type as a template. The resulting 1715-bp fragment contained a HindIII restriction site at the 5'-end, and a XhoI site at the 3'-end, and two mutations at bases 2253(C-T) and 2262 (G-T), generating premature translation stop codons in the 55-kD E1b reading frame at codons 79 and 82, respectively. The DNA fragment was digested with NheI/XhoI and cloned into the NheI/XhoI-digested pBS-(IRES) (Alexe V Gordadze et al. J. Virology 2001, 75:5899-5912) to generate pBS-IRES-E1A. The IRES-E1A DNA fragment was cut by SpeI/XhoI digestion to release fragment (IRES)-EIA. Human HSP70 cDNA was generated from cDNA of SKOV3 as described in Example 1. The DNA fragment obtained by the PCR contained a HindIII restriction site at the 5'-end, and a SpeI site at the 3'-end. Three pieces of DNA fragments: HindIII/SpeI-cut HSP70, SpeI/XhoI-cut IRES-E1A and HindIII/XhoI-cut pLEP were ligated to generate the desired pLEP-HSP-E1A (Fig. 4).

The resulted pLEP-HSP-E1A plasmid was then cut with Pi-PspI and ligated together with Pi-PspI-cut PREP at 16°C overnight. The ligated products were packaged with phage (Strategene Inc) and then incubated with E. coli. DK1 or DH5 in LB at 37°C for 30 minutes. The infected E. coli were plated on Ampicillin/Tetracycline agar plates, and colonies were screened and confirmed by restriction enzyme analysis to identify recombinant plasmid pAd-Hsp70/E1A. The pAd-Hsp70/E1A plasmid was then cut with I-CeuI and added to HEBS buffer, which contains 2.5M CaCl₂, to form DNA/Ca₃(PO₄)₂ mixture for transfection of 293 cells. After incubation in a CO₂ incubator for 6-7 days, clear plaques appeared on the cell lawn, indicating that recombinant adenoviruses were generated. When the plates were full of clear plaques, the cells were harvested by centrifugation for 10 minutes at 1500 rpm. The recombinant adenoviruses Ad-HSP/E1A were harvested after three repeated freeze-thaw cycles. The recombinant adenovirus titers were 10⁷-10⁸ PFU (Fig. 5).

### In Vitro Cytotoxicity and In Vivo Tumor Inhibition

The same procedures as described in Example 1 were used to test virus cytotoxicity in vitro and anti-tumor activity in mouse models. Consistent with the above experiment results, the recombinant adenoviruses expressing HSP70 were shown to have the ability to elicit potent immune response to inhibit distal tumors.

### Example 3

### Generation of Salmonella typhimurium Expressing HSP

[109] Salmonella typhimurium SL3261^{[19]} contains an aroA mutation, which results in the selective growth and killing of tumor cells, since only tumor cells, not normal cells, can provide the nutrients for the bacteria to grow. The plasmid pcDNA-HSP was generated by cloning human HSP70 cDNA into pcDNA3.1 under the control of the CMV promoter. The expression driven by the CMV promoter is strong but transient.

### Anti-tumor Activity

Syngeneic BALB/c mice were used to evaluate the anti-tumor activity of the recombinatnt Salmonella typhimurium SL3261 expressing HSP. Mice were divided into three groups: groups 1 and 2 were for therapeutic tests and the third group was for control. SMMC7721 tumor cells (Shanghai Tumor Collection Center), 1x10⁵, were injected subcutaneously into the bilateral flanks of mice. Those mice received inoculation of SMMC7721 tumor cells were regarded as animal model suffering from metastatic tumors. When subcutaneous tumors were palpably growing (about 5 mm in maximal diameter), each animal of Group 1 then underwent a unilateral intratumoral inoculation of recombinant SL3261 containing pcDNA-HSP (1x10⁷ PFU) in the right flank tumor, followed by a second inoculation 7 days later. Each animal of Group 2 then underwent a unilateral intratumoral inoculation of SL3261 (1x10⁶ PFU) in the right flank tumor, followed by a second inoculation 7 days later. The SL3261 used in Group 2 was used as a control for the recombinant SL3261 inoculation so that differences in bacterial factors would be accounted for. To evaluate the effect of the HSP per se, each animal of Group 3 then underwent a unilateral intratumoral inoculation of HSP in the right flank tumor, followed by a second inoculation 7 days later. Tumor size was measured by external caliper, and tumor volume was calculated (V = h x w x d). If animals appeared moribund or the diameter of their subcutaneous tumors reached 18 mm, they were killed and this was recorded as the date of death for survival studies. Statistical differences were calculated using StatView 4.5 (Abacus Concepts, Berkeley, CA) where mean tumor volume was assessed by unpaired t test. Inoculation with Salmonella typhimurium SL3261 containing pcDNA-HSP elicited a prominent anti-tumor effect, with both the inoculated tumors and their non-inoculated contralateral counterparts demonstrating a significant reduction in tumor growth (Fig. 7). Inoculation with Salmonella typhimurium only resulted in a significant reduction in tumor growth of inoculated tumors, but had less effect in noninoculated tumor (Fig. 7). This result indicates that treatment with Salmonella typhimurium SL3261 containing pcDNA-HSP elicits potent anti-tumor immune responses to block distal tumor growth.

The disclosure of the following references is hereby incorporated by references into this application.

### References

1. Kim D, Martuza RL, Zwiebel J. Replication-selective virotherapy for cancer: Biological principles, risk management and future directions. Nat Med. 2001;7:781-7.
2. Kim DH, McCormick F. Replicating viruses as selective cancer therapeutics. Mol Med Today. 1996;2:519-27.
3. Kim D, Hermiston T, McCormick F. ONYX-015: clinical data are encouraging. Nat Med. 1998;4:1341-2.
4. Matzinger P. An innate sense of danger. Semin Immunol. 1998;10:399-415.
5. Hartl FU. Molecular chaperones in cellular protein folding. Nature. 1996;381:571-9.
6. Udono H, Levey DL, Srivastava PK. Cellular requirements for tumor-specific immunity elicited by heat shock proteins: tumor rejection antigen gp96 primes CD8+ T cells in vivo. Proc Natl Acad Sci U S A. 1994;91:3077-81.
7. Udono H, Srivastava PK. Heat shock protein 70-associated peptides elicit specific cancer immunity. J Exp Med. 1993;178:1391-6.
8. Li Z, Srivastava PK. Tumor rejection antigen gp96/grp94 is an ATPase: implications for protein folding and antigen presentation. EMBO J. 1993;12:3143-51.
9. Heise CC, Williams A, Olesch J, Kim DH. Efficacy of a replication-competent adenovirus (ONYX-015) following intratumoral injection: intratumoral spread and distribution effects. Cancer Gene Ther. 1999;6:499-504.
10. Heise CC, Williams AM, Xue S, Propst M, Kim DH. Intravenous administration of ONYX-015, a selectively replicating adenovirus, induces antitumoral efficacy. Cancer Res. 1999;59:2623-8.
11. Oliff A, Gibbs JB, McCormick F. New molecular targets for cancer therapy. Sci Am. 1996;275:144-9.
12. Hall AR, Dix BR, O'Carroll SJ, Braithwaite AW. p53-dependent cell death/apoptosis is required for a productive adenovirus infection. Nat Med. 1998;4:1068-72.
13. Dix BR, O'Carroll SJ, Myers CJ, Edwards SJ, Braithwaite AW. Efficient induction of cell death by adenoviruses requires binding of E1B55k and p53. Cancer Res. 2000;60:2666-72.
14. Rogulski KR, Freytag SO, Zhang K, Gilbert JD, Paielli DL, Kim JH, Heise CC, Kim DH. In vivo antitumor activity of ONYX-015 is influenced by p53 status and is augmented by radiotherapy. Cancer Res. 2000;60:1193-6.
15. Kwong A, Frenkel N. Biology of herpes simplex virus (HSV) defective viruses and development of the amplicon system. In: Viral Vectors: Gene Therapy and Neuroscience Application. Eds: Kaplitt MG, Loewy, AD. 1995. New York, Academic Press, pp.25-42.
16. Mineta T, Rabkin SD, Yazaki T, Hunter WD, Martuza RL. Attenuated multi-mutated herpes simplex virus-1 for the treatment of malignant gliomas. Nat Med. 1995;1:938-43.
17. Spaete RR, Frenkel N. The herpes simplex virus amplicon: a new eucaryotic defective-virus cloning-amplifying vector. Cell. 1982;30:295-304.
18. Kaplitt MG, Pfaus JG, Kleopoulos SP, Hanlon BA, Rabkin SD, Pfaff DW. Expression of a functional foreign gene in adult mammalian brain following in vivo transfer via a herpes simplex virus type 1 defective viral vector. Mol Cell Neurosci. 1991;2:320-330.
19. Brown A, Hormaeche CE, Demarco de Hormaeche R, Winther M, Dougan G, Maskell DJ, Stocker BA. An attenuated aroA Salmonella typhimurium vaccine elicits humoral and cellular immunity to cloned beta-galactosidase in mice. J Infect Dis. 1987;155:86-92.
20. Pawelek JM, Low KB, Bermudes D. Tumor-targeted Salmonella as a novel anticancer vector. Cancer Res. 1997;57:4537-44.
21. Theys J, Landuyt W, Nuyts S, Van Mellaert L, van Oosterom A, Lambin P, Anne J. Specific targeting of cytosine deaminase to solid tumors by engineered Clostridium acetobutylicum. Cancer Gene Ther. 2001;8:294-7.

## Claims

1. An oncolytic microorganism that expresses a protein with the ability to chaperon antigenic peptides of tumor cells to antigen-presenting cells (APCs), and that selectively replicates in and kills tumor cells.

2. The oncolytic microorganism of claim 1, wherein said protein with the ability to chaperon antigenic peptides of tumor cells to APCs is heat shock protein (HSP) or a variant thereof.

3. The oncolytic microorganism of claim 2, wherein said HSP is from mammalian animal or microorganisms.

4. The oncolytic microorganism of claim 3, wherein said HSP is of human origin, and wherein said human HSP includes Hsp70, Hsp90, Hsp94, Hsp96, or other HSP.

5. The oncolytic microorganism of claim 3, wherein said HSP is of pathogen origin, including *Mycoplasma tuberculosis, Mycoplasma leprae, Trypanosoma cruzi, and Plasmodium falciparum*.

6. The oncolytic microorganism of claim 1, wherein said oncolytic microorganism is an oncolytic virus.

7. The oncolytic microorganism of claim 2, wherein said oncolytic microorganism is an oncolytic virus.

8. The oncolytic microorganism of claim 6 or 7, wherein said oncolytic virus includes adenovirus, herpes simplex virus (HSV), vesiculovirus, Newcastle disease virus, reovirus and other oncolytic viruses that can selectively replicate in tumor cells.

9. The oncolytic microorganism of claim 8, wherein said oncolytic virus is an adenovirus.

10. The oncolytic microorganism of claim 8, wherein said oncolytic virus is a HSV.

11. The oncolytic microorganism of claim 1 or 2, wherein said oncolytic microorganism is a bacterium.

12. The oncolytic microorganism of claim 11, wherein said bacterium is one that can selectively replicate in tumor cells, including Salmonella, Bifidobacterium, Shigella, Listeria, Yersinia, and Clostridium.

13. An APC transformed with a vector that contains a DNA sequence encoding a protein or fragment thereof that can chaperon antigenic peptides of tumor cells to APCs, wherein said vector can contain a DNA sequence encoding an immune-enhancing molecule as well.

14. The APC of claim 13, wherein said protein or fragment thereof that can chaperon antigenic peptides of tumor cells to APCs is HSP or a variant thereof.

15. A microorganism composition, including:
a) an oncolytic microorganism that can selectively replicate in and lyse tumor cells; and
b) a vector that can express a protein and fragment thereof that can chaperon antigenic peptides of tumor cells to APCs;
wherein said oncolytic microorganism contains an additional DNA sequence encoding an immune-enhancing molecule.

16. The microorganism composition of claim 15, wherein said protein and fragment thereof that can chaperon antigenic peptides of tumor cells to APCs is HSP or a variant thereof.

17. The microorganism composition of claim 15 or 16, wherein said oncolytic microorganism is an oncolytic virus or oncolytic bacterium.

18. The microorganism composition of claim 17, wherein said oncolytic virus includes adenovirus, HSV, vesiculovirus, Newcastle disease virus, reovirus and other oncolytic viruses that can selectively replicate in tumor cells.

19. The microorganism composition of claim 17, wherein said oncolytic bacteria include Salmonella, Bifidobacterium, Shigella, Listeria, Yersinia, and Clostridium that can selectively replicate in tumor cells.

20. The microorganism composition of claim 15, wherein said vector is a plasmid that contains a DNA sequence encoding the protein or fragment thereof that can chaperon antigenic peptides of tumor cells to APCs.

21. The microorganism composition of claim 15, wherein said vector is a replication-incompetent vector.

22. The microorganism composition of claim 20 or 21, wherein said protein or fragment thereof that can chaperon antigenic peptides of tumor cells to APCs is HSP or a variant thereof.

23. A pharmaceutical composition that mainly contains:
a) oncolytic microorganisms that can selectively replicate in tumor cells; and
b) a protein or fragment thereof that can chaperon antigenic peptides of tumor cells to APCs; and optionally
c) an immune-enhancement factor, immunological adjuvant, or pharmaceutical carrier,
wherein the immune-enhancement factor may be expressed by the oncolytic microorganism.

24. The pharmaceutical composition of claim 23, wherein said microorganisms are oncolytic viruses, including adenovirus, HSV, vesiculovirus, Newcastle disease virus, reovirus and other oncolytic viruses that can selectively replicate in tumor cells.

25. The pharmaceutical composition of claim 23, wherein said microorganisms are oncolytic bacteria including Salmonella, Bifidobacterium, Shigella, Listeria, Yersinia, and Clostridium that can selectively replicate in tumor cells.

26. The pharmaceutical composition of any one of claims 23-25, wherein said protein or fragment thereof that can chaperon antigenic peptides of tumor cells to APCs is HSP or a variant thereof.

27. A cancer immunotherapeutic agent, comprising the oncolytic microorganism according to any one of claims 1-12; or the APCs according to claim 13 or 14; or the microorganism composition according to any one of claims 15-22; or the pharmaceutical composition according to any one of claims 23-26.

28. The cancer immunotherapeutic agent of claim 27, further comprising an immune-enhancement factor, immunological adjuvant, or pharmaceutical carrier.

29. A method of tumor therapy, comprising transfection of APCs of the tumor patient with a vector that contains a DNA sequence encoding a protein or fragment thereof that can chaperon antigenic peptides of tumor cells to APCs.

30. The method of tumor therapy of claim 29, wherein said protein or fragment thereof that can chaperon antigenic peptides of tumor cells to APCs is HSP or a variant thereof.

31. A method of tumor therapy, comprising administration to a cancer patient of the oncolytic microorganism according to any one of claims 1-12; the APCs according to claim 13 or 14; the microorganism composition according to any one of claims 15-22, the pharmaceutical composition according to any one of claims 23-26, or the immunotherapeutic agent according to claims 27 or 28.

32. The method of tumor therapy of claims 29-31, wherein the tumors include benign and malignant tumors.

33. The method of claim 32, wherein said malignant tumors include melanoma, breast, prostate, hepatocellular, lung, nasopharyngeal, colon, ovarian, cervical tumors, and lymphoma.

34. The treatment method of claim 32, wherein the administration is conducted through intratumor, intramuscular, intravenous, or intraperitoneal injection, or oral or rectal administration.

35. The use of the oncolytic microorganism according to any one of claims 1-12, the APCs according to claim 13 or 14, the microorganism composition according to any one of claims 15-22, the pharmaceutical composition according to any one of claims 23-26, or immunotherapeutic agent according to claim 27 or 28 in preparation of an anti-tumor medicine.


Sequence Listing

<110> CHEN, siyi
HU, fang
Hangzhou Conquer Biotech Co., Ltd.

<120> oncolytic microorganisms expressing Heat Shock Protein and us thereof

<130>

<140> pct
<141> 2001-10-08

<160> 3

<170> PatentIn Ver. 2.1

<210> 1
<211> 23
<212> DNA
<213> Artificial Sequence

<220>
<223> Description of the artificial sequence: primer

<400> 1
ggtatggaag atccctcgag atc 23

<210> 2
<211> 26
<212> DNA
<213> Artificial Sequence

<220>
<223> Description of the artificial sequence: primer

<400> 2
tactaatcta cctcctcaat ggtggg 26

<210> 3
<211> 10494
<212> DNA

<400> 3
gcggctgacc ggagcgtccg ttgggcgaca aacaccagga cggggcacag gtacactatc 60
gggccgccgg gtaaaagaag tgagaacgcg aagcgttcgc acttcgtccc aatatatata 120
tattattagg gcgaagtgcg agcactggcg ccgggatcca aatgagtctt cggacctcgc 180
gggggccgct taagcggtgg ttagggtttg tctgacgcgg ggggaggggg aaggaacgaa 240
acactctcat tcggaggcgg ctcggggttt ggtggttacc tggggctgtg cggttgggac 300

ggcgcccgtg ggcccgggcg gccgggggcg gcgggggccg gcatggcggc gcggcgggcc 360
atggagacag agagcgtgcc ggggtggtag agtttgacag gcaagcatgt gcgtgcagag 420
gcgagtagtg cttgcctgtc taactcgctc gtctcggccg cgggggggcc gggctgcgcc 480
gccgcgcttt aaagggccgc gcgcgacccc cggggggtgt gtttcggggg gggcccgttt 540
tcgctcctcc ccccgctcct ccccccgctc ctccccccgc tcctcccccc gctcctcccc 600
ccgctcctcc ccccgctcct ccccccgctc ctccccccgc tcctcccccc gctcctcccc 660
ccgctcctcc ccccgctcct ccccccgctc ctccccccgc tcctcccccc gctcctcccc 720
ccgctcctcc ccccgctcct ccccccgctc ctccccccgc tcctcccccc gctcctcccc 780
gctcccgcgg ccccgccccc aacgcccgct cctccccgct cccgcggccc cgcccccaac 840
gcccgctcct ccccgctccc gcggccccgc ccccaacgcc cgccgcgcgc gcgcacgccg 900
cccggaccgc cgcccgcctt ttttgcgcgc cgccccgccc gcggggggcc cgggctgcca 960
caggtgtaac aacaccaaca gaacaccaac agcacggcgc accggcgact ccggttcctc 1020
atccacacgt cacacgtcac gtcatccacc acacctgccc accaacacaa ctcacagcga 1080
caacacaccg cgcaacaact cctgttcctc atccacacgt caccgcgcac cccccgctcc 1140
tccagacgtc ccccagcgca acacgccgct cctgtcacac accaccgccc cagccctccc 1200
cagccccagc cctccccagc cccagccctc cccagcccca gccctcccca gccccagccc 1260
tccccagccc cagccctccc cagccccagc cctccccagc cccagccctc cccagcccca 1320
gccctcccca gccccagccc tccccagccc cagccctccc cagccccagc cctccccagc 1380
cccagccctc cccagcccca gccctcccca gccccagccc tccccagccc cagccctccc 1440
cagccccagc cctccccagc cccagccctc cccagcccca gccctcccca gcgcgtcccg 1500
cgctccctcg ggggggttcg ggcatctcta cctcagtgcc gccaatctca ggtcagagat 1560
ccaaaccctc cggcgctgga cgacggggga gctggtgctc gggtgcgatg ccgcgctggt 1620
gcgcgcgcgc tacgggcggg aattcttgaa gacgaaaggg cctcgtgata cgcctatttt 1680
tataggttaa tgtcatgata ataatggttt cttagacgtc aggtggcact tttcggggaa 1740
atgtgcgcgg aacccctatt tgtttatttt tctaaataca ttcaaatatg tatccgctca 1800
tgagacaata accctgataa atgcttcaat aatattgaaa aaggaagagt atgagtattc 1860
aacatttccg tgtcgccctt attccctttt ttgcggcatt ttgccttcct gtttttgctc 1920
acccagaaac gctggtgaaa gtaaaagatg ctgaagatca gttgggtgca cgagtgggtt 1980
acatcgaact ggatctcaac agcggtaaga tccttgagag ttttcgcccc gaagaacgtt 2040
ttccaatgat gagcactttt aaagttctgc tatgtggcgc ggtattatcc cgtgttgacg 2100
ccgggcaaga gcaactcggt cgccgcatac actattctca gaatgacttg gttgagtact 2160
caccagtcac agaaaagcat cttacggatg gcatgacagt aagagaatta tgcagtgctg 2220
ccataaccat gagtgataac actgcggcca acttacttct gacaacgatc ggaggaccga 2280
aggagctaac cgcttttttg cacaacatgg gggatcatgt aactcgcctt gatcgttggg 2340
aaccggagct gaatgaagcc ataccaaacg acgagcgtga caccacgatg cctgcagcaa 2400
tggcaacaac gttgcgcaaa ctattaactg gcgaactact tactctagct tcccggcaac 2460
aattaataga ctggatggag gcggataaag ttgcaggacc acttctgcgc tcggcccttc 2520
cggctggctg gtttattgct gataaatctg gagccggtga gcgtgggtct cgcggtatca 2580
ttgcagcact ggggccttat ttgtgaaatt tgtgatgcta ttgctttatt cttggtggcc 2640
acgggcacgc agaagagcgc cgcgatcctc ttaagcaccc ccccgccctc cgtggaggcg 2700
ggggtttggt cggcgggtgg taactggcgg gccgctgact cgggcgggtc gcgcgcccca 2760
gagtgtgacc ttttcggtct gctcgcagac ccccgggcgg cgccgccgcg taaagccgcc 2820
gggggtccgt gtcctgtctc ggttggccgg acccgggccc gagcccgagc agtagccggc 2880
gccatgtcgg tggtgggcat agacctgggc ttccagagct gctacgtcgc tgtggcccgc 2940
gccggcggca tcgagactat cgctaatgag tatagcgacc gctgcacgcc ggcttgcatt 3000
tcttttggtc ctaagaatcg ttcaattgga gcagcagcta aaagccaggt aatttctaat 3060
gcaaagaaca cagtccaagg atttaaaaga ttccatggcc gagcattctc tgatccattt 3120
gtggaggcag aaaaatctaa ccttgcatat gatattgtgc agtggcctac aggattaaca 3180
ggtataaagg tgacatatat ggaggaagag cgaaatttta ccactgagca agtgactgcc 3240
atgcttttgt ccaaactgaa ggagacagcc gaaagtgttc ttaagaagcc tgtagttgac 3300
tgtgttgttt cggttccttg tttctatact gatgcagaaa gacgatcagt gatggatgca 3360

acacagattg ctggtcttaa ttgcttgcga ttaatgaatg aaaccactgc agttgctctt 3420
gcatatggaa tctataagca ggatcttcct cgcttagaag agaaaccaag aaatgtagtt 3480
tttgtagaca tgggccactc tgcttatcaa gtttctgtat gtgcatttaa tagaggaaaa 3540
ctgaaagttc tggccactgc atttgacacg acattgggag gtagaaaatt tgatgaagtg 3600
ttagtaaatc acttctgtga agaatttggg aagaaataca agctagacat taagtccaaa 3660
atccgtgcat tattacgact ctctcaggag tgtgagaaac tcaagaaatt gatgagtgca 3720
aatgcttcag atctcccttt gagcattgaa tgttttatga atgatgttga tgtatctgga 3780
actatgaata gaggcaaatt tctggagatg tgcaatgatc tcttagctag agtggagcca 3840
ccacttcgta gtgttttgga acaaaccaag ttaaagaaag aagatattta tgcagtggag 3900
atagttggtg gtgctacacg aatccctgcg gtaaaagaga agatcagcaa atttttcggt 3960
aaagaactta gtacaacatt aaatgctgat gaagctgtca ctcgaggctg tgcattgcag 4020
tgtgccatct tatcgcctgc tttcaaagtc agagaatttt ctatcactga tgtagtacca 4080
tatccaatat ctctgagatg gaattctcca gctgaagaag ggtcaagtga ctgtgaagtc 4140
ttttccaaaa atcatgctgc tcctttctct aaagttctta cattttatag aaaggaacct 4200
ttcactcttg aggcctacta cagctctcct caggatttgc cctatccaga tcctgctata 4260
gctcagtttt cagttcagaa agtcactcct cagtctgatg gctccagttc aaaagtgaaa 4320
gtcaaagttc gagtaaatgt ccatggcatt ttcagtgtgt ccagtgcatc tttagtggag 4380
gttcacaagt ctgaggaaaa tgaggagcca atggaaacag atcagaatgc aaaggaggaa 4440
gagaagatgc aagtggacca ggaggaacca catgttgaag agcaacagca gcagacacca 4500
gcagaaaata aggcagagtc tgaagaaatg gagacctctc aagctggatc caaggataaa 4560
aagatggacc aaccacccca atgccaagaa ggcaaaagtg aagaccagta ctgtggacct 4620
gccaatcgag aatcagctat atggcagata gacagagaga tgctcaactt gtacattgaa 4680
aatgagggta agatgatcat gcaggataaa ctggagaagg agcggaatga tgctaagaac 4740
gcagtggagg aatatgtgta tgaaatgaga gacaagctta gtggtgaata tgagaagttt 4800
gtgagtgaag atgatcgtaa cagttttact ttgaaactgg aagatactga aaattggttg 4860
tatgaggatg gagaagacca gccaaagcaa gtttatgttg ataagttggc tgaattaaaa 4920
aatctaggtc aacctattaa gatacgtttc caggaatctg aagaacgacc aaattatttg 4980
aagaactaga gaacttaggg aaacagatcc aacagtatat gaaaataatc gcggcaacgg 5040
gctcgctggg tcctaggctc aatggggacc gtatacgtgg acaggctctg gagcatccgc 5100
acgactgcgg tgatattacc ggagaccttc tgcgggacga gccgggtcac atctgacggt 5160
tcactaaacc agctctgctt atatagacct cccaccgtac acgcctaccg cccatttgcg 5220
tcaatggggc ggagttgtta cgacattttg gaaagtcccg ttgattttgg tgccaaaaca 5280
aactcccatt gacgtcaatg gggtggagac ttggaaatcc ccgtgagtca aaccgctatc 5340
cacgcccatt gatgtactgc caaaaccgca tcaccatggt aatagcgatg actaatacgt 5400
agatgtactg ccaagtagga aagtcccata aggtcatgta ctgggcataa tgccaggcgg 5460
gccatttacc gtcattgacg tcaatagggg gcgtacttgg catatgatac acttgatgta 5520
ctgccaagtg ggcagtttac cgtaaatact ccacccatga cgtcaatgga aagtccctat 5580
tggcgttact atgggaacat acgtcattat tgacgtcaat gggcgggggt cgttgggcgg 5640
tcagccaggc gggccattta ccgtaagtta tgtaacgcgg aactccatat atgggctatg 5700
aactaatgac cccgtaattg attactatta caatacgccc gcgtttcttc cttttcccca 5760
ccccaacccc caagttcggg tgaaggccca gggctcgcag ccaacgtcgg ggcggcaagc 5820
cctgccatag ccacgggccc cgtgggttag ggcgccccga ctgcatctgc gtgttcgaat 5880
tcgccaatga caagacgctg ggcggggttt gtgtcatcat agaactaaag acatgcaaat 5940
atatttcttc cggggacacc gccagcaaac gcgagcaacg ggccacgggg atgaagcagc 6000
ggccgagttt ccgcgggacc ccggccagct gctttacatc ccgaagacct acctgctcgg 6060
ccggcccccg aacgcgagcc tgcccgcccc caccacggtc gagccgaccg cccagcctcc 6120
cccctcggtc gcccccctta agggtctctt gcacaatcca gccgcctccg tgttgctgca 6180
tggcttcgta cccctgccat caacacgcgt ctgcgttcga ccaggctgcg cgttctcgcg 6240
gccatagcaa ccgacgtacg gcgttgcgcc ctcgccggca acaagaagcc acggaagtcc 6300
gcctggagca gaaaatgccc acgctactgc gggtttatat agacggtcct cacgggatgg 6360
ggaaaaccac caccacgcaa ctgctggtgg ccctgggttc gcgcgacgat atcgtctacg 6420

tacccgagcc gatgacttac tggcgggtgc tgggggcttc cgagacaatc gcgaacatct 6480
acaccacaca acaccgcctc gaccagggtg agatatcggc cggggacgcg gcggtggtaa 6540
tgacaagcgc ccagataaca atgggcatgc cttatgccgt gaccgacgcc gttctggctc 6600
ctcatatcgg gggggaggct gggagctcac atgccccgcc cccggccctc accctcatct 6660
tcgaccgcca tcccatcgcc gccctcctgt gttacccggc cgcgcgatac cttatgggca 6720
gcatgacccc ccaggccgtg ctggcgttcg tggccctcat cccgccgacc ttgcccggca 6780
caaacatcgt gcttggggcc cttccggagg acagacacat cgaccgcctg gccaaacgcc 6840
agcgccccgg cgagcggctt gacctggcta tgctggctgc gattcgccgc gtttacgggc 6900
tacttgccaa tacggtgcgg tatctgcagt gcggcgggtc gtggcgggag gactggggac 6960
agctttcggg gacggccgtg acgccccagg gtgccgagcc ccagagcaac gcgggcccac 7020
gaccccatat cggggaaacg ttatttaccc tgtttcgggc ccccgagttg ctggccccca 7080
acggcgacct gtacaacgtg tttgcctggg ccttggacgt cttggccaaa cgcctccgtc 7140
ccatgcacgt ctttatcctg gattacgacc aatcgcccgc cggctgccgg gacgccctgc 7200
tgcaacttac ctccgggatg gtccagaccc atgtcaccac cccaggctcc ataccgacga 7260
tctgcgacct ggcgcgcacg tttgcccggg agatggggga ggctcactga ggacggggtc 7320
ccccatgggg aatggtttat ggttcgtggg ggttattatt ttgggcgttg cgtggggtca 7380
ggtccacgac tggactgagc agacagaccc atggtttttg gatggcctgg gcatggaccg 7440
catgtactgg cgcgacacga acaccgggcg tctgtggctg ccaaacaccc ccgaccccca 7500
aaaaccaccg cgcggatttc tggcgccgcc ggacgaacta aacctgacta cggcatctct 7560
gccccttctt cgctggtacg aggagcgctt ttgttttgta ttggtcacca ggggtctgac 7620
gctcagtgga acgaaaactc acgttaaggg attttggtca tgagattatc aaaaaggatc 7680
ttcacctaga tccttttaaa ttaaaaatga agttttaaat caatctaaag tatatatgag 7740
taaacttggt ctgacagtta ccaatgctta atcagtgagg cacctatctc agcgatctgt 7800
ctatttcgtt catccatagt tgcctgactc cccgtcgtgt agataactac gatacgggag 7860
ggcttaccat ctggccccag tgctgcaatg ataccgcgag acccacgctc accggctcca 7920
gatttatcag caataaacca gccagccgga agggccgagc gcagaagtgg tcctgcaact 7980
ttatccgcct ccatccagtc tattaattgt tgccgggaag ctagagtaag tagttcgcca 8040
gttaatagtt tgcgcaacgt tgttgccatt gctgcaggca tcgtggtgtc acgctcgtcg 8100
tttggtatgg cttcattcag ctccggttcc caacgatcaa ggcgagttac atgatccccc 8160
atgttgtgca aaaaagcggt tagctccttc ggtcctccga tcgttgtcag aagtaagttg 8220
gccgcagtgt tatcactcat ggttatggca gcactgcata attctcttac tgtcatgcca 8280
tccgtaagat gcttttctgt gactggtgag tactcaacca agtcattctg agaatagtgt 8340
atgcggcgac cgagttgctc ttgcccggcg tcaacacggg ataataccgc gccacatagc 8400
agaactttaa aagtgctcat cattggaaaa cgttcttcgg ggcgaaaact ctcaaggatc 8460
ttaccgctgt tgagatccag ttcgatgtaa cccactcgtg cacccaactg atcttcagca 8520
tcttttactt tcaccagcgt ttctgggtga gcaaaaacag gaaggcaaaa tgccgcaaaa 8580
aagggaataa gggcgacacg gaaatgttga atactcatac aataccggaa ggaacccgcg 8640
ctatgacggc aataaaaaga cagaataaaa cgcacgggtg ttgggtcgtt tgttcataaa 8700
cgcggggttc ggtcccaggg ctggcactct gtcgataccc caccgagacc ccattgggac 8760
gttcccgggc ctgggtaacg ttttcggccg tccctgaccc cgaggccctg acgttcccgc 8820
ggggagacaa cgtggcgacg gcgagccacc cgagcgggcc gcgtgataca ccgccccccc 8880
gaccgccggt tggggcccgg cggcacccga cgacggagct ggacatcacg cacctgcaca 8940
acgcgtccac gacctggttg gccacccggg gcctgttgag atccccaggt aggtacgtgt 9000
atttctcccc gtcggcctcg acgtggcccg tgggcatctg gacgacgggg gagctggtgc 9060
tcgggtgcga tgccgcgctg gtgcgcgcgc gctacgggcg ggaattcttg aagacgaaag 9120
ggcctcgtga tacgcctatt tttataggtt aatgtcatga taataatggt ttcttagacg 9180
tcaggtggca cttttcgggg aaatgtgcgc ggaaccccta tttgtttatt tttctaaata 9240
cattcaaata tgtatccgct catgagacaa taaccctgat aaatgcttca ataatattga 9300
aaaaggaaga gtatgagtat tcaacatttc cgtgtcgcct gacattttcg gtgcgagtat 9360
ccgtaccatt cagaactggc aggaacaggg aatgcccgtt ctgcgaggcg gtggcaaggg 9420
taatgaggtg ctttatgact ctgccgccgt cataaaatgg tatgccgaaa gggatgctga 9480

aattgagaac gaaaagctgc gccgggagcc cggcggcacc tcgctaacgg attcaccact 9540
ccaagaattg gagccaatca attcttgcgg agaactgtga atgcgcaaac caacccttgg 9600
cagaacatat ccatcgcgtc cgccatctcc agcagccgca cgcggcgcat ctcggggccg 9660
acgcgctggg ctacgtcttg ctggcgttcg cgacgcgagg ctggatggcc ttccccatta 9720
tgattcttct cgcttccggc ggcatcggga tgcccgcgtt gcaggccatg ctgtccaggc 9780
aggtagatga cgaccatcag ggacagcttc aaggatcgct cgcggctctt accagcgcca 9840
gcaaaaggcc aggaaccgta aaaaggccgc gttgctggcg tttttccata ggctccgccc 9900
ccctgacgag catcacaaaa atcgacgctc aagtcagagg tggcgaaacc cgacaggact 9960
ataaagatac caggcgtttc cccctggaag ctccctcgtg cgctctcctg ttccgaccct 10020
gccgcttacc ggatacctgt ccgcctttct cccttcggga agcgtggcgc tttctcatag 10080
ctcacgctgt aggtatctca gttcggtgta ggtcgttcgc tccaagctgg gctgtgtgca 10140
cgaacccccc gttcagcccg accgctgcgc cttatccggt aactatcgtc ttgagtccaa 10200
cccggtaaga cacgacttat cgccactggc agcagccact ggtaacagga ttagcagagc 10260
gaggtatgta ggcggtgcta cagagttctt gaagtggtgg cctaactacg gctacactag 10320
aaggacagta tttggtatct gcgctctgct gaagccagtt accttcggaa aaagagttgg 10380
tagctcttga tccggcaaac aaaccaccgc tggtagcggt ggtttttttg tttgcaagca 10440
gcagattacg cgcagaaaaa aaggatctca agaagatcct ttgatctttt ctac 10494